# EUROPÄISCHE PATENTANMELDUNG

(11) **EP 0 957 174 A1**
(43) Veröffentlichungstag der Anmeldung: **17.11.1999**
(21) Anmeldenummer: 99108790.9
(22) Anmeldetag: 03.05.1999
(51) Int. Cl.: C12P 17/12, C07D 239/06, C07B 63/00, B01D 61/14

(54) **Verfahren zur Auftrennung von Tetrahydropyrimidinderivaten**

(30) Priorität: 13.05.1998 DE 19821378
(71) Anmelder: Degussa-Hüls Aktiengesellschaft, 60287 Frankfurt am Main (DE)
(72) Erfinder: Karau, Andreas Dr., 52134 Herzogenrath (DE); Treffenfeldt, Wiltrud Prof., 63179 Obertshausen (DE); Preuss, Andrea Dr., 63456 Hanau (DE); Stockhammer, Stefan, 97662 Brusno (SK)

(57) **Zusammenfassung**

Die Erfindung betrifft ein Verfahren zur Auftrennung von Tetra-hydropyrimidin-Derivaten, insbesondere 2-Methyl-1,4,5,6-tetrahydropyrimidin-4-Carbonsäuren (Ectoine)und 2-Methyl,5-Hydroxyl-1,4,5,6-tetrahydropyrimidin-4-Carbonsäuren (Hydroxyectoine), die nebeneinander in wäßrigen Lösungen, insbesondere Fermentationsbrühen, vorliegen, durch selektive Adsorption einer Komponente an Zeolithen.

## Beschreibung

Die Erfindung betrifft ein Verfahren zur Auftrennung von Tetra-hydropyrimidin-Derivaten, insbesondere 2-Methyl-1,4,5,6-tetrahydropyrimidin-4-Carbonsäuren (Ectoine)und 2-Methyl, 5-Hydroxyl-1,4,5,6-tetrahydropyrimidin-4-Carbonsäuren (Hydroxyectoine), die nebeneinander in wäßrigen Lösungen vorliegen, durch selektive Adsorption einer Komponente an Zeolithen.

Mit Tetrahydropyrimidinderivaten sind insbesondere die aus der EP-A1-0553884 bekannten Verbindungen gemeint.

Strukturell sind Ectoine cyclische Aminosäurederivate, die zur Klasse der sogenannten 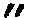 kompatiblen Solute" gehören. Sie sind auch in hoher Konzentration mit dem Cytoplasma kompatibel und stabilisieren die Zellkomponenten in einem Milieu geringer Wasseraktivität. Diese Wirkung weist auf ein breites Anwendungsfeld in medizinischen und kosmetischen Bereichen hin.

Durch neue biotechnische Verfahren ist es gelungen, halophile Eubakterien z. B. der Gattung Halomonas zu züchten und diese Organismen zum Ausscheiden der Ectoine in das sie umgebende Medium zu veranlassen (T. Sauer et al. GIT Fachz. Lab. 10/95).

Da in Abhängigkeit von den Fermentationsbedingungen sowohl Ectione wie auch Hydroxyectoine produziert werden, ist in den nachfolgenden Aufarbeitungsschritten eine Trennung der beiden Komponenten erforderlich, um die Reinkomponenten zu erhalten. Diese Trennung kann z.B. durch die Extraktion mit Methanol unter Ausnutzung der unterschiedlichen Löslichkeiten von Ectoin und Hydroxyectoin erfolgen (*T. Sauer und Erwin A. Galinski. Biotechnology and Bioengineering, VOL 57, NO 3, 1998*). Dieses Verfahren ist jedoch im technischen Maßstab durch die erheblichen Mengen an benötigtem Methanol aufwendig.

Aufgabe der Erfindung ist es, ein alternatives Verfahren zur Verfügung zu stellen, das eine effektive Trennung von Tetrahydropyrimidinderivaten, insbesondere Ectoine und Hydroxyectoine, aus einer gegebenenfalls weitere organische und anorganische Verbindungen enthaltenen wäßrigen Lösung ohne Einsatz von Lösungsmitteln ermöglicht.

Gegenstand der Erfindung ist ein Verfahren zur Abtrennung von in wäßriger Lösung vorliegenden Tetrahydropyrimidinen, von den entsprechenden Hydroxyverbindungen, insbesondere von 2-Methyl-1,4,5,6-Tetrahydropyrimidin-4-Carbonsäuren (Ectoine (I)) 2-Methyl, 5-Hydroxyl-1,4,5,6-tetrahydropyrimidin-4-Carbonsäuren (Hydroxyectoine) (II)die in wäßrigen Lösungen nebeneinander vorliegen, dadurch gekennzeichnet, daß man die wäßrigen Lösungen, welche beide Komponenten enthalten, bei einem pH-Wert von 1,5 bis 7,0 mit einem bevorzugt sauren Zeolith in Kontakt bringt, der einen Modul von 15 bis 1000 besitzt. Hierbei erfolgt eine kinetisch bevorzugte Adsorption von Ectoin gegenüber Hydroxyectoin. Durch die optimale Einstellung der experimentell zu ermittelnden Kontaktzeit zwischen Zeolith und Lösung, erfolgt eine selektive Abreicherung der Ausgangslösung an Ectoin, während das Hydroxyectoin zunächst in Lösung bleibt und erst bei längeren Kontaktzeiten in signifikanter Menge adsorbiert wird. Die am Zeolith gebundenen Ectoin-Derivate werden durch die Einstellung des pH-Werts auf >8,0, insbesondere unter Verwendung einer Ammoniumhydroxidlösung, von dem eingesetzten Zeolithen desorbiert. Man kann die Lösung auch durch Zusatz einer anderer basischer, gebenenfalls organischer Komponenten, insbesondere Lysin, auf diesen pH-Wert einstellen.

In Abhängigkeit von der notwendigen Reinheit der zu isolierenden Derivate ist das Reinigungsverfahren gegebenenfalls in mehrfach aufeinander folgenden Schritten durchzuführen.

Unter den erfindungsgemäß zu trennenden Tetrahydropyrimidinderivaten versteht man insbesondere Verbindungen der Formel 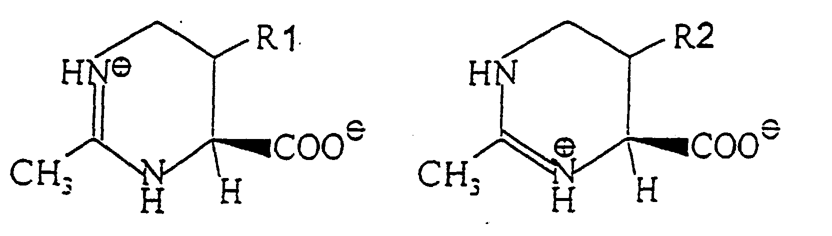 mit der Bedeutung für R1: H und R2: OH.

Die Lösungen, aus denen man diese Stoffe abtrennt, sind im allgemeinen wäßriger oder organischer Natur.

Für die Adsorption der erfindungsgemäß abzutrennenden Verbindungen geeignete Zeolithe sind solche der Typen, Y, DAY, Mordenit, dealuminierter Mordenit, ZSM-5, dealuminierter ZSM-5, β oder VPI5 und einem Modul von 10 bis 1000, insbesondere von 15 bis 200, bevorzugt von 15 bis 45. Bevorzugt setzt man den ZSM-5-Typ der H-, Ammonium- oder Na-Form ein.

Das Verfahren läuft im allgemeinen in einem Temperaturbereich zwischen 15 und 80°C ab, bevorzugt 20 bis 40 ° C.

Die Konzentrationen der aufzutrennenden Ectoine bzw. der Hydroxyextoine bewegen sich im bekannten Löslichkeitsbereich. Die aus dem Stand der Technik geläufigen Konzentrationsverhältnisse (molar) der aufzutrennenden Verbindungen sind mit dem erfindungsgemäßen Verfahren aufarbeitbar und liefern das gewünschten Produkt in der notwendigen Reinheit.Die Adsorbentien werden pulverförmig, als Formkörper oder trägerfixiert eingesetzt. Für die verfahrenstechnische Realisierung stehen das Batch-, Festbett oder Crossflow-Filtrationsverfahren kontinuierlich oder diskontinuierlich durchgeführt zur Verfügung.

In einer vorteilhaften Ausführungsform kombiniert man die Adsorption an Zeolith mit einer Crossflow-Filtration , bei der man eine gegebenenfalls von der Biomasse befreite, die Tetrahydropyrimidinderivate enthaltende Lösung mit der Suspensionen eines sauren Zeoliths für eine der Adsorptionskinetik entsprechenden Zeitintervall in Kontakt bringt und anschließend
die im Überstand vorhandenen Hydroxy-Tetrapyrmidinderivate über die Crossflow-Filtration abtrennt, in dem man
   a) die beladenen Zeolithe in Form von Suspensionen an einer porösen Fläche/Membran vorbeiströmen läßt, wobei man
   b) eine Druckdifferenz zwischen der Überström- und der gegenüberliegenden Seite der Fläche/Membran einstellt, so daß
   c) ein Teil der die Fläche/Membran überströmenden von den adsorbierten Verbindungen ganz oder teilweise befreiten die Hydroxyverbindungen enthaltenden Lösung die Fläche/Membran quer zur Fließrichtung durchströmt (Filtratfluß)
   d) in einem Waschschritt die von den adsorbierten Verbindungen befreite Lösung, die die Hydroxyverbindungen enthält, abtrennt, und
   e) anschließend die adsorbierten Verbindungen desorbiert.

Letzteres geschieht bei einem geeigneten pH-Wert, insbesondere bei einem pH-Wert >8,0.

Als Adsorbentien besonders geeignet sind feinteilige Zeolithpulver der oben genannten Typen mit einem Partikeldurchmesser von 1 bis 100 µm, insbesondere 2 bis 20 µm. Die kurzen intrapartikulären Diffusionswege ermöglichen hohe Selektivitäten bei der Adsorption und damit hohe Trennfaktoren.

Die erreichten Reinheiten für Hydroxyectoin im Überstand (bezogen auf Gesamtectoin) liegen bei Verwendung von z.B. ZSM5/28 nach einer Kontaktzeit von 1 h bei 100% (Beispiel 1). Bei Mordenit 30 liegen die Reinheiten aufgrund einer langsameren Kinetik nach 24 h bei ca. 60% (Beispiel 2). Sie können jedoch bei einer geeigneten Kombination der Verfahrensschritte gesteigert werden.

Zur Erhöhung der Reinheit schleust man die Lösungen ein- oder mehrfach in das erfindungsgemäße Reinigungsverfahren ein. Eine Variante des erfindungsgemäßen Verfahrens besteht darin, dieses mit aus dem Stand der Technik bekannten Reinigungsverfahren durch Verwendung von organischen Kationenaustauschern und anderen Reinigungsschritten zu kombinieren.

Ein entsprechender Adsorptions- und Desorptionsschritt erfolgt vor und/oder nach einer Reinigungsstufe durch Adsorption an einem der erfindungsgemäß verwendeten Zeolithe, der sich eine Desorption angeschlossen hat.

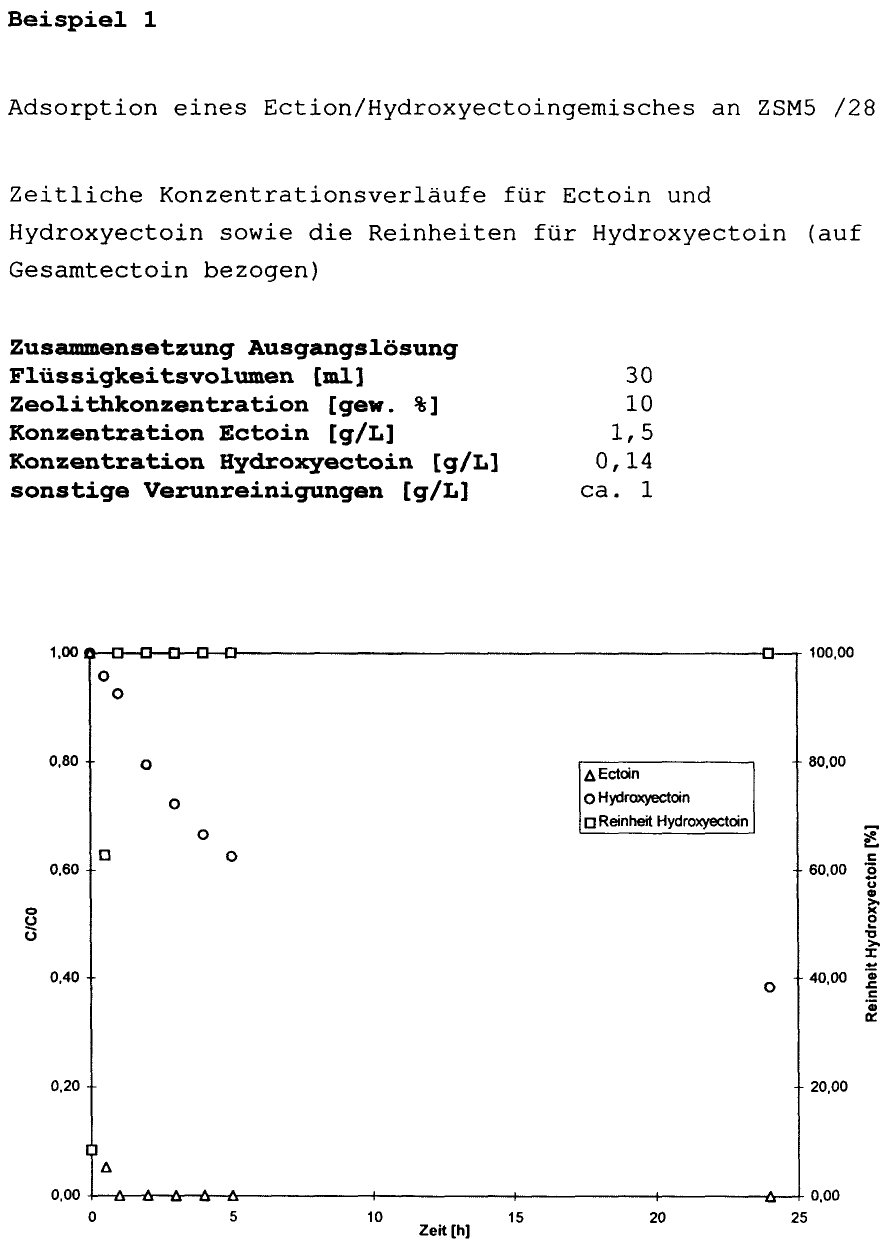

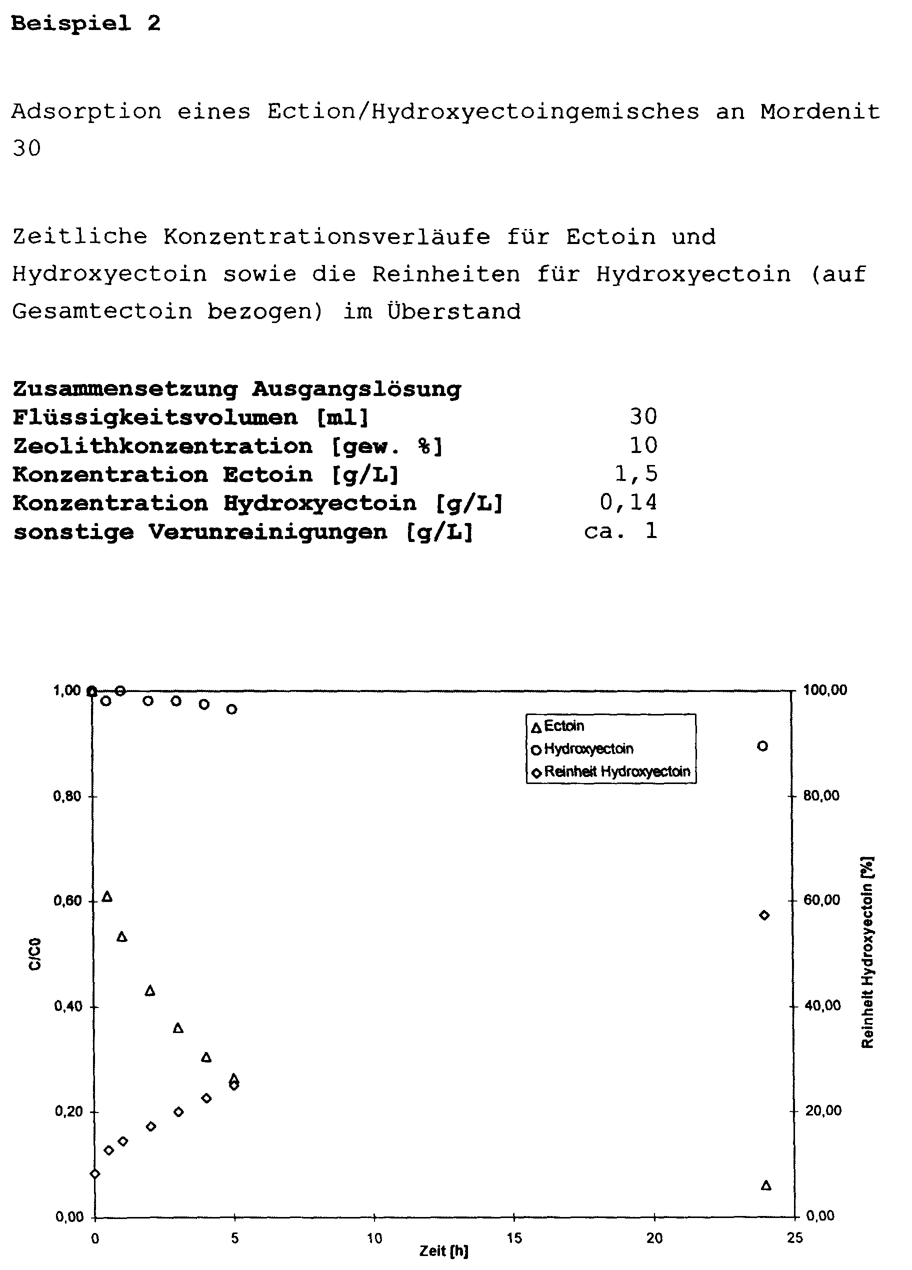

## Patentansprüche

1. Verfahren zur Abtrennung von in wäßriger Lösung vorliegenden Tetrahydropyrimidinen von den entsprechenden Hydroxyverbindungen,
**dadurch gekennzeichnet,**
daß man die wäßrigen Lösungen dieser Verbindungen bei einem pH-Wert von 1,0 bis 7,0 mit einem sauren Zeolith in Kontakt bringt, der einen Modul von 15 bis 1000 besitzt, nach erfolgter Adsorption den bevorzugt die Hydroxyverbindungen enthaltenden Überstand abtrennt und die adsorbierten Derivate mit einer wäßrigen, auf einen pH Wert >8,0 gegebenfalls durch Zusatz einer basischen organischen Komponente eingestellten Lösung, von den Zeolithen desorbiert.

2. Verfahren gemäß Anspruch 1,
**dadurch gekennzeichnet**,
daß man ein in wäßriger Lösung vorliegendes Gemisch von 2-Methyl-1,4,5,6-Tetrahydropyrimidin-4-Carbonsäuren (Ectoine) der allgemeinen Formel 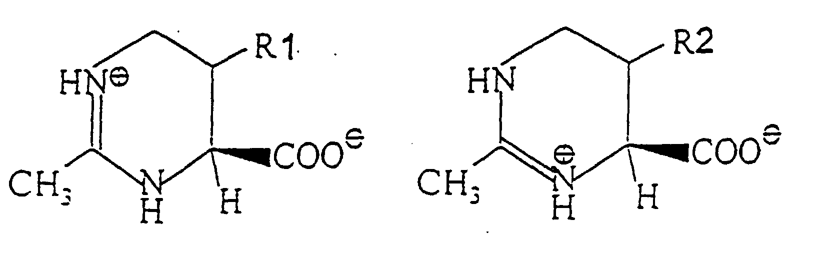 mit der Bedeutung für R1:H und R2:OH auftrennt.

3. Verfahren gemäß den Ansprüchen 1 und 2
**dadurch gekennzeichnet,**
daß man den nach der Adsorption erhaltenen Überstand erneut mit einem der oben genannten Zeolithtypen in Kontakt bringt, das Adsorbens anschließend abtrennt und diese Schritte gegebenfalls ein- oder mehrfach wiederholt.

4. Verfahren gemäß den Ansprüchen 1 bis 3,
**dadurch gekennzeichnet,**
daß man als wäßrige Lösung eine Fermentationslösung einsetzt.

5. Verfahren gemäß Anspruch 4,
**dadurch gekennzeichnet,**
daß man vor der Adsorption die Mikroorganismen zumindest teilweise aus der Fermentationslösung abtrennt.

6. Verfahren gemäß den Ansprüchen 4 und 5,
**dadurch gekennzeichnet,**
daß man vor der Adsorption die löslichen Proteine zumindest teilweise aus der Fermentationslösung abtrennt.

7. Verfahren gemäß einem oder mehreren der Ansprüche 1 bis 6,
**dadurch gekennzeichnet,**
daß man als Adsorbentien saure Zeolithe der Typen Y, DAY, Mordenit, dealuminierter Mordenit, ZSM-5, dealuminierter ZSM-5, β oder VPI-5 mit dem Modul von 15 bis 1000 einsetzt.

8. Verfahren gemäß Anspruch 6,
**dadurch gekennzeichnet,**
daß man Zeolithe vom Typ ZSM5 oder Mordenit in der H-, Ammonium oder Na-Form einsetzt.

9. Verfahren gemäß einem oder mehreren der Ansprüche 1 bis 8 zur Auftrennung der oben bezeichneten Tetrahydropyrimidinderivate, insbesondere gemäß Formel (I),
**dadurch gekennzeichnet,**
daß man es mit einer Crossflow-Filtration kombiniert, wobei man
a) die beladenen Zeolithe in Form von Suspensionen an einer porösen Fläche/Membran vorbeistromen läßt, wobei man
b) eine Druckdifferenz zwischen der Überström- und der gegenüberliegenden Seite der Fläche/Membran einstellt, so daß
c) ein Teil der die Fläche/Membran überströmenden von den adsorbierten Verbindungen ganz oder teilweise befreiten die Hydroxyverbindungen enthaltenden Lösung die Fläche/Membran quer zur Fließrichtung durchströmt (Filtratfluß)
d) in einem Waschschritt die von den adsorbierten Verbindungen befreite Lösung, die die Hydroxyverbindungen enthält, abtrennt, und
e) anschließend die adsorbierten Verbindungen desorbiert.

10. Verfahren gemäß Anspruch 9,
**dadurch gekennzeichnet,**
daß man Zeolithe mit einem mittleren Partikeldurchmesser von 1 bis 100 µm einsetzt.

11. Verfahren gemäß den Ansprüchen 9 und 10,
**dadurch gekennzeichnet,**
daß man einen Transmembrandruck von 0,2 bis 3 bar einstellt.

12. Verfahren gemäß den Ansprüchen 9 bis 11,
**dadurch gekennzeichnet,**
daß man keramische oder organische Membranen/poröse Flächen mit Ultrafiltrations- oder Mikro- oder Nanofiltrationseigenschaften verwendet.

13. Verfahren gemäß einem oder mehreren der vorhergehenden Ansprüche,
**dadurch gekennzeichnet,**
daß man die desorbierte Lösung ein- oder mehrfach der Adsorption und Desorption unterwirft.

14. Verfahren gemäß einem oder mehreren der vorhergehenden Ansprüche,
**dadurch gekennzeichnet,**
daß man das Verfahren zur Auftrennung von Verbindungen (A) mit der Adsorption an Kationenaustauschern oder anderen Reinigungsschritten und den daraus durch Desorption gegebenfalls wiederholt gewonnenen Lösungen kombiniert.
